# EUROPEAN PATENT SPECIFICATION

(11) **EP 2 554 179 B1**
(45) Date of publication and mention of the grant of the patent: **15.01.2020**
(21) Application number: 12179357.4
(22) Date of filing: 06.08.2012
(51) Int. Cl.: A61K 36/185, A61K 8/00, A61K 9/00, A61P 17/06

(54) **Composition based on a vegetable extract for the treatment of cutaneous inflammatory forms, in particular psoriasis.**
Zusammensetzung, basierend auf einem Gemüseextrakt für die Behandlung von kutanen Entzündungsformen, insbesondere Psoriasis
Composition à base d'un extrait végétal pour le traitement des formes inflammatoires cutanées, en particulier le psoriasis

(30) Priority: 04.08.2011 IT MI20111494
(43) Date of publication of application: 06.02.2013
(73) Proprietor: 1&18 USA LLC, Miami Beach, FL 33139 (US)
(72) Inventor: Asuni, Giambattista, 09020 USSANA (IT)
(74) Representative: Coppo, Alessandro

(56) References cited:
- WO-A1-2005/034900
- WO-A2-2010/084496
- US-A1- 2008 199 489

## Description

### FIELD OF THE INVENTION

The present invention concerns a composition based on a vegetable extract for the treatment of cutaneous inflammatory forms, in particular psoriasis.

The present invention originates in the phytotherapeutic and/or cosmetic product sector.

More specifically, the present invention relates to a composition containing biologically active components of vegetable origin, which finds application in the topical treatment of psoriasis and of inflammatory skin conditions.

### BACKGROUND OF THE INVENTION

Psoriasis is an inflammatory skin condition, which is generally chronic and often recurrent, that affects around 1-2% of the world's population.

Psoriasis affects men and women in equal measure although there are subtypes of the condition in which there is a prevalence of one of the two sexes.

In the cutaneous areas affected by psoriasis there is an excessively rapid development of psoriasis there is an excessively rapid development of keratinocytes, which do not succeed in completing their maturation and differentiation function and in forming the mature stratum corneum of the skin that determines the barrier effect of the skin. Psoriasis therefore manifests with well defined erythematous papules or plaques that are red in colour, covered with dry, white or silver scales that are sometimes associated with itching and burning. The lesions are of various dimensions and the severity can range from a few sites of dandruff-type desquamation to general dermatosis with arthritis (psoriatic arthritis), exfoliations and debilitating rashes. When the condition progresses pustulous lesions may also appear.

Psoriatic lesions can affect the whole body but are more frequently localized at the level of the elbows, the knees, the scalp and the umbilical and sacral areas.

Psoriatic lesions are presented at histological level as areas of hyperproliferation, with an epidermal turnover that I accelerated by around 10 times with respect to normal skin, with an incomplete maturation of the keratocytes and maintenance of the nuclei in the stratum corneum (parakeratosis), with tortuous neovascularization, increase in blood flow, protein exudates and immature lymphatic vessels The aetiology of psoriasis is not yet known however available data seems to indicate that it is a condition of multi-factor origin that is genetically, clinically and histologically heterogeneous.

The is evidence of the implication of genetic factors in the disease since there is an increased incidence of psoriasis in individuals with HLA-CW6 and HLA-DR7, even if gene pathways appear to be rather low and gene expression irregular. There is also evidence that dysregulation of the cell-mediated immunity compartment (T-cells) and of the epidermal response to certain stimuli plays a fundamental role in the genesis and maintenance of this disorder. In particular, it seems that there are various factors that unleash T-cell dysregulation.

It is believed that there are risk factors that can predispose to development of the condition.

There are also several conditions that, while not classified as risk factors, can be the eliciting cause of psoriasis in subjects that are deeded as being predisposed; these conditions are therefore defined as eliciting factors.

One typical, albeit non-exclusive, characteristic of psoriasis is the so-called Koebner phenomenon, which consists of the appearance of dermatological lesions at the site of physical trauma such as a scratch, a burn, a scar, etc.

The treatment for psoriasis is generally of the local type while the systemic treatment is reserved for the most serious forms, affecting large parts of the body. The systemic treatment includes phototherapy with UVB, or with UVA and psoralens, photochemotherapy or PUVA treatment. Corticosteroids, retinoids or cyclosporine A may also be administered in some cases.

The local treatment of psoriasis envisages the local application of topical preparations containing emollient substance such as petrolatum, or containing anti-inflammatory substances such as cortisone preparations, reducing agents such as tar, dithranol or keratolytic agents such as salicylic acid and/or urea or, even, Vitamin D analogues.

However, to date, the available treatments have not provided a satisfactory solution for the disease as such treatments only result in complete remission of the symptoms of the disease in a small percentage of cases.

In addition, since in the local treatment the most pharmacologically active ingredients such as the cortisone preparations, the vitamin D analogues are applied on large areas of the affected skin, in which the protection/barrier function of the skin is compromized, there is systemic absorption of these medicines with the appearance of secondary effects that can also be significant in cases in which the treated areas are particularly extensive.

The International patent application WO 2010/084496 A2 discloses a composition comprising chard, beta vulgaris ssp. cicla, for the topical treatment of psoriasis and other dermatological diseases.

The patent application US 2008/199489 A1 discloses a composition comprising chard for the topical treatment of psoriasis and other dermatoses.

The International patent application WO 2005/034900 A1 discloses the use of sugar beet extract (Beta vulgaris), for the topical treatment of wrinkles and other ageing symptoms as well as for post-traumatic therapy of abrasions, cuts, burns and gum injuries.

In the current state of the art, there is therefore a need to make available new preparations that are able to provide a higher degree of remission of the disease and of the cutaneous lesions connected thereto, without determining an increase in the secondary effects.

One of the main objects of the present invention therefore consists of providing a composition for the treatment of psoriasis or of inflammatory skin conditions of the of vegetable origin and that are therefore almost free of side effects when applied to the affected skin or administered by oral route.

### SUMMARY OF THE INVENTION

The present invention originates from the discovery that vegetable extracts of beet, a herbaceous plant of the Chenopodiaceae family, contain one or more biologically active components that find application in the treatment of inflammatory diseases of the skin, in particular if attributable to psoriasis.

According to a first aspect of the present invention, it is provided a composition for use as defined in the appended claim 1.

A beet plant suitable within the scope of the invention is a herbaceous plant that is widely diffused in temperate zones, with bright green leaves of large dimensions and with white, red or yellow stalks, depending on the type.

Specifically, the beet plant utilized within the scope of the invention is of the order of the Caryophyllales, belongs to the Chenopodiaceae family, to the genus Beta, Species: B. vulgaris, subspecies, in particular *cicla* variety (*Beta Cicla*). Beet is further identified by the following common names, in English: Swiss chard, silverbeet, perpetual spinach, spinach beet, crab beet, bright lights, seakale beet and mangold.

In certain embodiments, the composition comprising the beet plant extract finds application in the therapeutical treatment of psoriasis or cutaneous manifestations thereof or in the symptomatology of dermatitides, particularly on an immunogenetic basis, such as Lichen ruber planus.

In certain embodiments, the disclosed composition comprises one vegetal component that is biologically active in the treatment of psoriasis, extracted from beet and a physiologically acceptable carrier.

A method is also disclosed for the treatment of psoriasis and/or of inflammations of the skin comprising the administration of a composition based on a beet plant extract to a body region in need of treatment.

According to other aspects, the treatment of the affections or cutaneous manifestations, in particular psoriasis are disclosed.

In accordance to an aspect the present invention provides a composition for use according to claim 7.

The use according to this aspect of the invention is specifically suitable for treating irritated skin or irritative cutaneous manifestation of the skin.

The term irritative cutaneous manifestations of the skin includes skin irritation conditions such as reddening, excessive flaking, hyperpigmentation or hyperkeratinization of the skin.

In certain embodiments, the cosmetically acceptable carrier of the composition of the invention is an excipient, carrier or diluent suitable for cosmetic formulations.

### DETAILED DESCRIPTION OF THE INVENTION

The Applicant has discovered that by mixing or placing in contact a vegetable matrix/portion of beet plant with a suitable solvent, the solvent enriches with one or more substances or ingredients that are biologically active in treating psoriasis or the skin manifestations thereof or inflammatory conditions or reddening of the skin, in general.

The present invention is therefore directed at the use of a vegetable extract of beet plant as an agent in the pharmaceutical or cosmetic treatment of psoriasis and extends to its use in the treatment of the dermatological affections or manifestations associated with psoriasis and/or inflammatory skin conditions.

The methods of the present disclosure provide an effective treatment of psoriasis and the skin manifestations thereof at a low cost, since the beet plant cultivations are very common and extended over wide areas such that the ingredients of the composition can be obtained at low costs.

In addition, the compositions for use according to claim 1 make use of components of vegetable origin that are biologically active and safe when administered by oral or local route.

In certain embodiments, the composition for use according to claim 1 comprising the vegetable extract of beet plant is administered to a subject, typically a mammal, specifically a human being. In certain embodiments, the composition is administered by oral route. In other embodiments, the composition is applied directly to the skin in need of treatment.

In certain embodiments, the composition for use of the invention is a medical device, or a pharmaceutical composition for oral or topical administration.

According to certain embodiments, the vegetable extract is derived from the roots, leaves, fruits or flowers of beet plant or from two or more of these parts of the plant. In certain embodiments, the vegetable extract is derived from the aerial portion of the beet plant. This type of extract has proved to be particularly effective in the applications according to the invention.

As described hereinbelow, the vegetable extract is extracted from the beet plant utilising a physiologically acceptable or edible solvent.

The term "edible" means a physiologically acceptable solvent that can be assimilated by the human body.

A suitable solvent for obtaining the vegetable extract is a physiologically acceptable and/or edible liquid in which the components that are biologically active in treating the inflammation of the skin are soluble and in which they are not subject to alteration that deprives them of activity.

A suitable solvent is hydrophilic and is selected from water, ethanol or a mixture thereof. Another solvent is lipophilic and typically comprises a fat such as a vegetable oil, such as olive, corn, sunflower, almond oil or mixtures thereof. In certain embodiments, the solvent can be inedible, in particular it can be distanced by evaporation, such as for example methanol, an ethyl ether, dimethyl ether for example.

As disclose herein, the composition comprises an aqueous extract from the beet plant.

In certain embodiments, the vegetable extract of beet plant and/or the composition that contains it according to this invention, is non-ionized.

In certain embodiments, the vegetable extract of plant beet has a pH above 6.6 and in certain embodiments the pH is substantially neutral or slightly basic pH, for example in the 7 to 8 range.

In certain embodiments, the composition for use of the invention has a pH above 6.6, typically basic or slightly alkaline for example of between 7 and 8. In certain embodiments, the pH of between 7 and 8 is obtained without the addition of buffer agents or other agents that control the pH of the composition.

In certain embodiments, the compositions for use of the invention have an amount of vegetable extract of beet above 10% p/w, for example of 10 to 80% by weight, typically of 15 to 70% by weight.

Typically, the vegetable extract of beet plant can be obtained by solid-liquid extraction techniques which are suitable for separating/extracting from the vegetable tissues of the beet plant one or more substances or ingredients which are biologically active in treating inflammatory skin diseases, particularly cutaneous affections associated with psoriasis.

As disclosed herein, the extraction of one or more biologically active components takes place by maceration of a vegetable portion or matrix of beet in a suitable solvent.

As disclosed herein, a vegetable portion or matrix of beet plant is immersed in a suitable solvent for a suitable time to enrich the solvent with one or more biologically active components. Under these conditions, the extraction in the solvent of the biologically active components present in the vegetable tissues of the plant takes place by diffusion and osmosis.

In the maceration, the matrix of beet plant is typically kept in contact with the solvent for a variable time to obtain the extraction of an effective amount of biologically active component. In certain embodiments, the maceration time can range between 10 and 72 hours.

The vegetable extract can be used as is to make wet packs on the areas of the body affected by psoriasis or it can be used in the formulation of pharmaceutical or cosmetic compositions for treating psoriasis or inflammatory conditions of the skin. Typically the extract can be fluid, soft or dry. For example, in the fluid extract 1 ml of extract contains biologically active components that are soluble in 1 g of herbal substance, in the soft extract the solvent is partially evaporate in particular until the extract wets the filter paper, in the dry extract the solvent is almost completely evaporated to obtain a powder.

In certain embodiments, the extraction takes place by utilising a weight ratio between the solvent and the vegetable matrix of between 1:10 and 10:1.

In certain embodiments, the extraction of the biologically active component of the vegetable matrix of beet is achieved utilising an oil-based solvent as extraction liquid.

Disclosed herein is a double extraction in the hydrophilic and lipophilic phase from the beet matrix. There is an extraction with a hydrophilic solvent, typically water, to extract the active hydrophilic components from the vegetable matrix of beet and then an extraction with a lipophilic solvent, typically a vegetable oil, to extract the active lipophilic components from the vegetable matrix of beet. The order of the two hydrophilic/lipophilic extractions can be inverted.

In certain embodiments, the vegetable extract from beet plant is obtained by
i) macerating a vegetable matrix of beet plant in a water-based solvent to extract the biologically active, water-soluble components,
ii) macerating the vegetable matrix of beet plant in a fat-based or oil-based solvent to extract the biologically active, fat-soluble components,
iii) combining the extract of phase i) with the extract of phase ii).

In certain embodiments, phases i) and ii) are carried out on the same vegetable matrix. In these embodiments, the order of the extraction phases i) and ii) can be inverted.

In certain embodiments, the aqueous extract is combined with the oil-based extract to produce formulations suitable for pharmaceutical or cosmetic uses.

The compositions for use of the invention contain a mixture of beet plant extracts in oil phase and in aqueous phase.

For example, the aqueous extract can be combined with the oil-based extract in a ratio from 10:1 to 1:5 by weight.

In certain embodiments the compositions for topical application are in the form of creams or gels containing excipients such as jellying agents and/or rheological modifiers which are conventional in the pharmaceutical or cosmetic field.

In certain embodiments, the combined aqueous and oil-based extract is utilized to prepare a cleansing shampoo to treat the scalp areas, by adding one or more conventional surface-active agents.

Further methods for obtaining the vegetable extract are well known by a person skilled in the art and include any methods used in the field of extractions from vegetable material. These include but are not limited to, extraction by means of solvent, digestion, infusion, pressing, decoction, percolation, countercurrent extraction, Soxhlet, extraction with super critical gases, ultrasounds, ASE™ (Accelerated Solvent Extraction by Dionex, USA).

In certain embodiments, the term composition comprising a beet plant extract extends to a composition comprising an analogue, precursor, metabolite, derivative, a synthesis version, or an active salt of the biologically active component of the extract of beet that maintains the same activity.

In certain embodiments, the biologically active component comprises a leukotriene or an amino acid or peptide containing L-proline.

In certain embodiments, the leukotriene is selected from 9,12,15-octadecatrienoic acid, 11,14,17-eicosatrienoic acid, their esters, in particular methyl and mixtures thereof.

In certain embodiments, the amino acid or peptide is L-Proline, 5-oxo-methyl ester.

In certain embodiments, the composition of the invention comprises a physiologically or pharmacologically acceptable carrier, diluent or excipient. This physiologically or pharmaceutically and/or cosmetically acceptable carrier, diluent or excipient can be selected on the basis of the route of administration for which the resulting pharmaceutical composition is intended.

In certain embodiments, the route of administration of the composition of the invention is the local route. Suitable formulations in solid form for local administration and for pharmaceutical or cosmetic uses include creams, gels, ointments, pastes, salves. In other embodiments, the formulation for local administration is in fluid or semi-fluid form. Suitable formulations in liquid or semi-fluid form include lotions, gels, shampoo suspensions, emulsions.

In the case of formulations in fluid or semi-fluid form, the extract can be diluted in a carrier in a physiologically acceptable liquid form such as water, alcohol, a hydroalcoholic or glycerol solution or mixed with other liquids suitable for local application.

In certain embodiments, the compositions of the invention comprises one or more excipients commonly utilized in the formulation of cosmetic or pharmaceutical preparations for local use, such as preservatives, agents, bactericides, stabilisers, emulsifiers, buffers, humectants, dyes and other excipients commonly used in cosmetic / pharmaceutical preparation techniques.

In one embodiment, the formulation for local application is in the form of emulsion containing the extract carried in a suitable excipient.

By way of example, suitable excipients are cellulose derivatives such as hydroxymethylcellulose, hydroxypropylmethylcellulose, methylcellulose, hydroxypropylcellulose, hydroxymethylcellulose, carboxyethylcellulose, ethylhydroxycellulose, cellulose acetate butyrate, and mixtures thereof.

Further examples of suitable excipients comprise the polymers that are part of the lactam family such as pyrrolidone and mixtures thereof, inorganic salts such as calcium or dicalcium phosphate, lubricants such as for example magnesium stearate, tryacylglycerols and mixtures thereof.

In certain embodiments, the composition for topical application comprises an excipient of the hydroxymethylcellulose type and/or Sepigel 305, and/or jellying agents with HLB suitable for the formulation and for the substances for example of 1.0 to 9.0.

According to other embodiments, the route of administration of the composition is oral. For the administration by oral route the vegetable extract is formulated in a suitable pharmaceutical or phytotherapeutic composition. The compositions for oral administration can be in solid or liquid form. Typical compositions in solid form comprise tablets, capsules, powders, granules, pills, solutions, emulsions, suspensions, syrups and controlled-release forms thereof.

Tablets generally comprise a suitable carrier or excipient in which the extract is dispersed, typically in dry form.

Typically, the biologically active components in the composition for use of the invention are present in variable quantities, for example of between 0.0001% by weight and 10% by weight, typically of between 00.1% and 5% by weight.

The amount administered and the frequency of administration of the composition will depend on the nature and gravity of the skin affections and lesions to be treated.

In certain embodiments, the vegetable extract of beet is administered at a concentration of between 0.1 and 100 mg/ml. The levels of concentration of vegetable extract of beet have proven suitable for obtaining an activity on the psoriasis or on irritative dermatological manifestations or dermatitides.

The present invention will now be described with reference to the following examples.

### EXAMPLES

### Example 1:

### Extraction method

The fresh vegetable matrix (whole beet aerial and root portion) 1 Kg is peeled and immersed in 1 Kg of deionized water and brought to the boil.

On boiling, the vegetable matrix (beet) is minced with the aid of a rotary blade mill, and left in extraction for at least 15 minutes, then cooled and filtered.

The filtrate is preserved with the addition of a mixture of antimicrobial preservatives suitable for cosmetic formulations, such as for example the mixture of Nipagin (methylparaben) and stored in stainless steel containers.

Deprived of the extraction water, the vegetable matrix is forced air-cooled and subsequently immersed in vegetable oil (such as corn, almond oil or mixtures of vegetable oils) at a 1:0.5 ratio.

The oil extraction continues for a duration of 10 days (in standard phase), or of 3 hours through the use of macerators for oleolites under pressure.

On completion of the extraction, the beet oleolite obtained is stored in stainless steel containers and then packaged in suitable bottles.

### Example 2

### Alternative extraction method

The fresh, recently gathered vegetable matrix (whole beet aerial and root portion) 2 Kg is peeled and immersed in 1 Kg of deionized water and brought to the boil. On boiling, the vegetable matrix (beet) is minced with the aid of a rotary blade mill, and left in extraction for at least 15 minutes, then cooled and filtered.

The filtrate is preserved with the addition of a mixture of classic antimicrobial preservatives for cosmetic formulations, such as Nipagin (methylparaben) and stored in stainless steel containers.

Deprived of the extraction water, the vegetable matrix is forced air-cooled and subsequently immersed in vegetable oil (such as corn, almond oil or mixtures of vegetable oils) at a 1:1 ratio.

The oil extraction continues for a duration of 10 days (in standard phase), or of 3 hours through the use of macerators for oleolites under pressure.

On completion of the extraction, the beet oleolite obtained is stored in stainless steel containers and then packaged into suitable bottles.

### Example 3

### Clinical trials on the action of extract of beet activity on psoriasis

18 volunteers affected by psoriasis (15 males, 3 females), one male volunteer affected by psoriasis and by Lichen ruber planus and one female volunteer affected by atopic dermatitis were treated daily with the hydrophilic solvent or with the lipophilic extract or with a formulation containing one or both extracts.

For the duration of the above-described treatment, all the volunteers made use of each botanical-cosmetic, pharmacological treatment.

The volunteers presented lesions typical of dermatitis in various areas of the body such as the elbow, knee, genitals, sole of the foot and the palm of the hands, the lumbosacral area, the scalp.

As early as 2-5 days into treatment, all the volunteers showed almost complete remission of the symptomatology and of the cutaneous manifestations typical of psoriasis (erythematous papules and plaques, desquamation, psoriatic arthritis and itching if present).

### Example 4

The extract obtained from example 1 was analysed by gas chromatography to check the content of the components having biological activity.

The main components of the extract resulting from the gas-mass chromatography are illustrated below in increasing order of the number of carbon atoms.

### 8 Carbon atoms

Octane, 2,4,6-trimethyl- Octane, 4-methyl-

### 10 Carbon atoms

1,10-Decanediol
4-Decyne
Decanedioic acid, didecyl ester

### 11 Carbon atoms

n-undecane-1,11-diol

### 12 Carbon atoms

1-Dodecyne
4-Decyne
1,2-Epoxy-1-vinylcyclododecene

### 14 Carbon atoms

Tetradecane
Tetradecanoic acid
1-Tetradecanol

### 15 Carbon atoms

Pentadecanoic acid
Pentadecanoic acid, ethyl ester
Pentadecane, 2-methyl-

### 16 Carbon atoms

Hexadecanoic acid
Hexadecanoic acid, ethyl ester
2-Hexadecen-1-ol, 3,7,11,15-tetram
Hexadecane, 1,1-dimethoxy
Hexadecane, 2-methyl- Hexadecane
2-Hexadecen-1-ol, 3,7,11,15-tetramethyl

### 17 Carbon atoms

Heptadecane
Heptadecane, 2-methyl- 1-Heptadecanol
1,6-Heptadiene, 2,5,5-trimethyl-

### 18 Carbon atoms

1-Octadecanol

### 9,12,15-Octadecatrienoic acid, met

### Mixed

Bis-(3,5,5-trimethylhexyl) ether
Bicyclo[4.3.0]nonane, 2-methylen-,

### 11,14,17-Eicosatrienoic acid, meth

3,8-Dioxatricyclo [5.1.0.0(2,4)]oct
Phenol
4H-Pyran-4-one, 2,3-dihydro-3,5-dihydroxy-6-methyl
2,4,5-trimethyl-1,3-dioxolane
Propane, 1-isothiocyanato- 1-Azabicyclo[2.2.2]octane, 4-ethyl

### L-Proline, 5-oxo-, methyl ester

3,3-Epoxymethano-6-(3'-isopropenylcyclopropen-1'-yl
1,2-Benzenedicarboxylic acid, bis(2-methylpropyl)

### Example 5

### Composition containing extract of beet in the form of gel cream

| | |
|---|---|
| AQUEOUS EXTRACT OF B. | 60 |
| HYDROXYETHYCELLULOSE | 2 |
| SEPIGEL 305 | 3 |
| POTASSIUM SORBATE | 0.20 |
| SODIUM BENZOATE | 0.15 |
| OLEOLITE OF B. | 30 |
| VIT. E | 1 |
| PURIFIED WATER Q.S. TO 100 | |

### Example 6

### Composition containing extract of beet in the form of shampoo

| | |
|---|---|
| AQUEOUS EXTRACT OF B. | 60 |
| TWEEN 80 | 20 |
| SODIUM LAURETH SULPHATE | 5 |
| OLEOLITE OF B. | 5 |
| HYDROXYETHYCELLULOSE | 3 |
| SODIUM BENZOATE | 0.15 |
| POTASSIUM SORBATE | 0.20 |
| PURIFIED WATER Q.S. TO 100 | |

### Example 7

### Composition containing extract of beet in the form of body wash

| | |
|---|---|
| AQUEOUS EXTRACT OF B. | 55 |
| TWEEN 80 | 25 |
| SODIUM LAURETH SULPHATE | 8 |
| OLEOLITE OF B. | 5 |
| HYDROXYETHYCELLULOSE | 4 |
| SODIUM BENZOATE | 0.15 |
| POTASSIUM SORBATE | 0.20 |
| PURIFIED WATER Q.S. TO 100 | |

## Claims

1. Composition comprising
a vegetable extract of a plant *Beta vulgaris, cicla* variety obtained by extracting the biologically active water-soluble components from matrix of *Beta vulgaris, cicla* variety with a water-based solvent and the biologically active liposoluble components with a lipophilic solvent and collecting the biologically active water-soluble and lipophilic components and
a physiologically acceptable carrier for use in the treatment of psoriasis and/or cutaneous inflammatory affections.

2. Composition for use according to Claim 1, wherein the vegetable extract of beet plant comprises a leukotrienic derivative selected from 9,12,15-octadecatrienoic acid, 11,14,17-eicosatrienoic acid, their esters and a L-Proline-containing amino acid.

3. Composition for use according to Claim 2, wherein said L-Proline-containing amino acid is L-Proline 5-oxo-methyl ester.

4. Composition for use according to any one of Claims 1-3, wherein the vegetable extract of beet plant is obtained by
i) macerating a vegetable matrix of beet plant in a water-based solvent to extract the biologically active, water-soluble components,
ii) macerating the vegetable matrix of beet plant in a fat-based or oil-based solvent to extract the biologically active, fat-soluble components,
iii) combining the extract of phase i) with the extract of phase ii).

5. Composition for use according to any one of Claims 1-4, **characterized in that** it is in a form for oral or topical administration.

6. Composition for use according to Claim 5, **characterized in that** it is in a form for topical application selected from cream, emulsion, ointment, shampoo or lotion.

7. Composition comprising a vegetable extract of a plant *Beta vulgaris, cicla* variety obtained by extracting the biologically active water-soluble components from matrix of *Beta vulgaris, cicla* variety with a water-based solvent and the biologically active liposoluble components with a lipophilic solvent and collecting the biologically active water-soluble and lipophilic components and a physiologically acceptable carrier, for use in the treatment of irritated skin.

8. Composition for use according to claim 7 wherein the irritated skin is reddening, excessive flaking, hyperpigmentation or hyperkeratinization of the skin.

9. Composition for use according to claim 7 wherein the composition is in the form of a water-based suspension or emulsion, and the physiologically acceptable carrier comprises an excipient based on a derivative of cellulose and/or a gelling agent.

## Patentansprüche

1. Zusammensetzung, Folgendes umfassend
einen Pflanzenextrakt aus der Pflanze *Beta vulgaris, var. cicla*, erhalten durch Extrahieren der biologisch aktiven wasserlöslichen Bestandteile aus der Matrix von *Beta vulgaris, var. cicla* mit einem wasserbasierten Lösungsmittel und der biologisch aktiven fettlöslichen Bestandteile mit einem lipophilen Lösungsmittel und Sammeln der biologisch aktiven wasserlöslichen und lipophilen Bestandteile und
einen physiologisch verträglichen Träger zur Verwendung bei der Behandlung von Psoriasis und/oder entzündlichen Hauterkrankungen.

2. Zusammensetzung zur Verwendung nach Anspruch 1, wobei der Pflanzenextrakt der Rübenpflanze ein Leukotrienderivat, ausgewählt aus 9,12,15-Octadecatriensäure, 11,14,17-Eicosatriensäure, deren Estern und eine L-Prolin enthaltende Aminosäure umfasst.

3. Zusammensetzung zur Verwendung nach Anspruch 2, wobei die L-Prolin enthaltende Aminosäure L-Prolin-5-Oxo-Methylester ist.

4. Zusammensetzung zur Verwendung nach einem der Ansprüche 1-3, wobei der Pflanzenextrakt der Rübenpflanze folgendermaßen erhalten wird:
i) Mazerieren einer pflanzlichen Rübenpflanzenmatrix in einem wasserbasierten Lösungsmittel, um die biologisch aktiven, wasserlöslichen Bestandteile zu extrahieren,
ii) Mazerieren der pflanzlichen Rübenpflanzenmatrix in einem fettbasierten oder ölbasierten Lösungsmittel, um die biologisch aktiven, fettlöslichen Bestandteile zu extrahieren,
iii) Kombinieren des Extrakts aus Phase i) mit dem Extrakt aus Phase ii).

5. Zusammensetzung zur Verwendung nach einem der Ansprüche 1-4, **dadurch gekennzeichnet, dass** sie in einer oralen bzw. topischen Darreichungsform vorliegt.

6. Zusammensetzung zur Verwendung nach Anspruch 5, **dadurch gekennzeichnet, dass** sie in einer topischen Darreichungsform vorliegt, ausgewählt aus Creme, Emulsion, Salbe, Shampoo oder Lotion.

7. Zusammensetzung, umfassend einen Pflanzenextrakt aus der Pflanze *Beta vulgaris, var. cicla*, erhalten durch Extrahieren der biologisch aktiven wasserlöslichen Bestandteile aus der Matrix von *Beta vulgaris, var. cicla* mit einem wasserbasierten Lösungsmittel und der biologisch aktiven fettlöslichen Bestandteile mit einem lipophilen Lösungsmittel und Sammeln der biologisch aktiven wasserlöslichen und lipophilen Bestandteile, und einen physiologisch verträglichen Träger zur Verwendung bei der Behandlung von gereizter Haut.

8. Zusammensetzung zur Verwendung nach Anspruch 7, wobei die gereizte Haut eine Rötung, übermäßige Abblätterung, Hyperpigmentierung oder Hyperkeratinisierung der Haut ist.

9. Zusammensetzung zur Verwendung nach Anspruch 7, wobei die Zusammensetzung in Form einer wasserbasierten Suspension oder Emulsion vorliegt, und der physiologisch verträgliche Träger einen Hilfsstoff auf Basis eines Derivats von Cellulose und/oder eines Geliermittels umfasst.

## Revendications

1. Composition comprenant
un extrait végétal de la plante *Beta vulgaris*, *de la variété cicla,* obtenue par extraction des composants hydrosolubles biologiquement actifs de la matrice de la *Beta vulgaris, de la variété cicla,* avec un solvant à base d'eau et des composants liposolubles biologiquement actifs avec un solvant lipophile, et récupération des composants hydrosolubles et lipophiles biologiquement actifs et
un support physiologiquement acceptable pour l'usage dans le traitement du psoriasis et/ou des affections inflammatoires cutanées.

2. Composition à utiliser selon la revendication 1, dans laquelle l'extrait végétal de betterave comprend un dérivé leucotriénique choisi parmi l'acide 9,12,15-octadécatriénoïque, l'acide 11,14,17-éicosatriénoïque, leurs esters et un acide aminé contenant de la L-proline.

3. Composition à utiliser selon la revendication 2, dans laquelle ledit acide aminé contenant de la L-proline est l'ester 5-oxo-méthylique de la L-proline.

4. Composition à utiliser selon l'une quelconque des revendications 1 à 3, dans laquelle l'extrait végétal de betterave est obtenu par :
i) macération d'une matrice végétale de betterave dans un solvant à base d'eau pour extraire les composants hydrosolubles biologiquement actifs,
ii) macération de la matrice végétale de betterave dans un solvant à base de graisse ou à base d'huile pour extraire les composants liposolubles biologiquement actifs,
iii) combinaison de l'extrait de la phase i) avec l'extrait de la phase ii).

5. Composition à utiliser selon l'une quelconque des revendications 1 à 4, **caractérisée en ce qu'**elle est sous une forme pour administration par voie orale ou topique.

6. Composition à utiliser selon la revendication 5, **caractérisée en ce qu'**elle est sous une forme pour application topique choisie parmi une crème, une émulsion, une pommade, un shampooing ou une lotion.

7. Composition comprenant un extrait végétal d'une plante *Beta vulgaris, de la variété cicla,* obtenue par extraction des composants hydrosolubles biologiquement actifs de la matrice de *Beta vulgaris, de la variété cicla* avec un solvant à base d'eau et des composants liposolubles biologiquement actifs avec un solvant lipophile, et par récupération des composants hydrosolubles et lipophiles biologiquement actifs et un support physiologiquement acceptable pour l'usage dans le traitement de la peaux irritée.

8. Composition à utiliser selon la revendication 7, dans laquelle la peau irritée est un rougissement, une desquamation excessive, une hyperpigmentation ou une hyperkératinisation de la peau.

9. Composition à utiliser selon la revendication 7, dans laquelle la composition est sous la forme d'une suspension ou une émulsion à base d'eau, et le support physiologiquement acceptable comprend un excipient à base d'un dérivé de cellulose et/ou d'un agent gélifiant.
